# EUROPEAN PATENT SPECIFICATION

(11) **EP 1 537 030 B1**
(45) Date of publication and mention of the grant of the patent: **13.05.2009**
(21) Application number: 03756373.1
(22) Date of filing: 03.06.2003
(51) Int. Cl.: B65D 51/16, B65D 47/24, C12L 9/00

(54) **VENTED CLOSURES FOR CONTAINERS**
BELÜFTETE VERSCHLÜSSE FÜR BEHÄLTER
FERMETURES VENTILEES POUR CONTENANTS

(30) Priority: 03.06.2002 US 388609 P
(43) Date of publication of application: 08.06.2005
(73) Proprietor: Advanced Porous Technologies, LLC, San Diego, CA 92124 (US)
(72) Inventor: SMOLKO, Daniel, D., Jamul, CA 91935 (US); KEVORKIAN, Gregory, Temecula, CA 92592 (US)
(74) Representative: Sevinç, Erkan
(86) International application number: PCT/US2003/017427
(87) International publication number: WO 2003/101858

(56) References cited:
- EP-A- 0 414 098
- EP-A- 0 414 098
- EP-A- 0 635 436
- EP-A- 0 635 436
- WO-A-93/18837
- FR-A- 2 736 329
- FR-A- 2 736 329
- US-A- 3 051 993
- US-A- 3 051 993
- US-A- 4 076 656
- US-A- 4 076 656
- US-A- 4 816 268
- US-A- 5 262 444
- US-A- 5 262 444
- US-A- 5 609 759
- US-A- 5 988 426
- US-A- 5 988 426
- US-A- 5 988 448
- US-A- 5 988 448
- US-A- 6 006 952
- US-A- 6 006 952
- US-A- 6 142 384
- US-A1- 2001 002 262
- US-A1- 2003 000 907
- US-B1- 6 299 038

## Description

### Field of the Invention

This invention relates to closures for beverage containers and more particularly to closures that are vented for the purpose of reducing negative pressure or vacuum that builds up inside the container when a beverage is being consumed therefrom.

### Description of the Related Art

A large variety of beverage containers are constructed with a small opening or drinking spout through which the fluid contents can be extracted. The opening is adapted so that a person can place their mouth over the opening thus forming a seal around the opening. Examples of these types of beverage containers include: a soda-pop bottle having a small annular opening; a drinking cup or spill-proof cup having a cover formed with a drinking spout; and, a nipple-equipped baby bottle. As the fluid contents are being consumed from one of these beverage containers, a negative pressure or vacuum builds up within the container making it necessary to interrupt drinking long enough to allow air to enter into the container equalizing the pressure between the outside and inside atmospheres. This interruption causes inconvenience for adult drinkers and makes it difficult for babies to continue feeding. Numerous solutions have been proposed whereby the beverage container is vented to relieve the buildup of negative pressure. As one would expect, most of the solutions are directed to spill-proof cups or baby bottles for feeding infants.

US 5,988,448 discloses a vacuum release container cap comprising a body, a closure device, and a seal member. The body has at least two passages formed therein, a liquid passage for the flow of liquid into and out of the container, and a gas passage for the flow of air. The closure device is mounted on the body for closing each of the passages. The closure device automatically opens the gas passage while opening the liquid passage. The seal member is mounted in the gas passage, which allows the passage of air into the container, but does not allow the passage of liquid out of the container though the gas passage whereby the disclosure of US 5,988,448 comprises inter alia seal members including porous vent material.

### Summary of the Invention

In accordance with the invention, there is provided a closure for dispensing fluids from a container as defined in claim 1. The closure comprises a pair of telescopically coupled first and second members cooperatively defining a fluid path, in which the first member is attached to a base or unitary with the base, the base is adapted to be secured, connected or attached to a container and the base and/or the first member include one or more sections of a porous vent material having at least two parts being in the form of a lamination or surface pattern which allows passage of gases and inhibits bulk passage of liquid. In a preferred embodiment, the fluid path is opened to allow fluid flow out of a container by moving the second member relative to the first member, including by twisting or pulling away the second member relative to the first. In some embodiments, the porous vent material is covered by the second member when the closure is in a closed position and exposed to air when the closure is in an open position.

Preferred embodiments of the closures and assemblies disclosed herein may include one or more of the following: a vent material comprising plastic, metal, ceramic and/or glass; hydrophobic vent material; and plastic vent material having a high water intrusion pressure. Additionally in preferred embodiments of closures and assemblies: the porous vent material provides sufficient venting to allow a substantially continuous liquid flux rate from the closure without creating a substantial pressure differential across the closure, preferably at least about 500 ml/min/cm², including at least about 50 ml/min/cm² ; the closure provides a pressure drop during dispensing of less than about 2 psi., including less than about 1 psi.

### Brief Description of the Drawings

Figure 1 is an exploded perspective view of a baby bottle showing the plastic bottle body, the vent, the nipple, and threaded ring in positional relationship to each other.
Figure 2A shows a cross section of the closed end of the bottle body showing the vent secured to the bottle body by injection molding (see line A, Figure 1 for plane of section for views 2A-2D and line B, Figure 1 for cut-off line defining the lower part of bottle in views 2a-2d).
Figure 2b shows a cross section of the closed end of the bottle body showing the vent secured to the bottle body by welding, sealant or sonic sealing.
Figure 2c is a cross-sectional side view of the closed end of the bottle body showing the vent formed as a plug and inserted into a hole formed in the bottle body.
Figure 2d is a cross-sectional side view of the closed end of the bottle body showing the vent formed as a plug with a shoulder and inserted into a cavity formed in the bottom of the bottle body.
Figure 3 is an exploded perspective view of a sports bottle with a vent shown in positional relationship to the bottom of the bottle.
Figure 4 is a cross-sectional side view of a screw-on lid for a drinking cup showing a vent secured to the inner surface of the cap by welding, sealant or sonic sealing.
Figures 5A through 5C depict various geometric arrangements of porous material in accordance with the invention within one or more planes.
Figures 6A and 6B show a stacked packaging configuration in exploded and side views of one embodiment of a telescopic vented bottle closure in accordance with the invention. This configuration is designed for use with packaging of carbonated beverages.
Figure 7 shows a multifunctional carbonated beverage closure system for pressure relief, venting, and fluid delivery.
Figures 8A and 8B show exploded views of multifunctional beverage closure system cap assembly, fluid, vent, and pressure relief paths.
Figures 9A through 9C show multifunctional beverage closure system cap position and engagement for pressure release, venting, and liquid release.

The figures illustrate various solutions and are intended to be merely exemplary. To that end, several figures contain optional features that need not be included in any particular embodiment of the invention, and the shape, type, or particular configuration of container or closure illustrated should not be taken as limiting on the invention, since the scope of the invention is defined by the claims.

### Detailed Description of the Preferred Embodiments

Disclosed herein below are beverage containers and container closures including those that are vented for the purpose of reducing negative pressure or vacuum that builds up inside the container when a beverage is being consumed therefrom. In preferred embodiments, the containers and/or closures comprise porous vent materials.

Porous vent materials may be made of any of a wide variety of materials, including, but not limited to, plastics, metals, glass, and ceramics. Combinations of plastics with metals, glass, or ceramics may also be used. The combinations may be intimate such as from blending of two or more components to become co-sintered, or may be layered such as from multilaminate structures derived from two or more materials. Combinations of different plastics, elastomers, metals, glasses, or ceramics can also be cosintered or fabricated into multilaminate structures for use as porous materials. Preferred plastics for porous vent materials include, but are not limited to thermoplastic polymers, thermoset elastomers, and thermoplastic elastomers. Preferred thermoplastic polymers include, but are not limited to, low density polyethylene (LDPE), linear low density polyethylene (LLDPE), medium density polyethylene (MDPE), high-density polyethylene (HDPE), ultra-high molecular weight polyethylene (UHMWPE), polypropylene (PP) and its copolymers, polymethylpentene (PMP), polybutylene terephthalate (PBT); polyethyleneterephthalate (PET), polyethyleneterephthalate glycol modified (PETG), polyetheretherketone (PEEK), ethylenevinylacetate (EVA), polyethylenevinylalcohol (EVOH), polyacetal, polyacrylonitrile (PAN), poly(acrylonitrile-butadiene-styrene) (ABS), poly(acrylonitrile-styrene-acrylate) (AES), poly(acrylonitrile-ethylene-propylene-styrene) (ASA), polyacrylates, polymethacrylates, polymethylmethacrylate (PMMA), polyvinylchloride (PVC), chlorinatedpolyvinylchloride (CPVC), polyvinyldichloride (PVDC) fluorinated ethylenepropylene (FEP), polyvinylfluoride (PVF), polyvinylidinefluoride (PVDF), polytetrafluoroethylene (PTFE), polyester, cellulosics, polyethylenetetrafluoroethylene (ETFE), polyperfluoroalkoxyethylene (PFA), nylon 6 (N6), polyamide, polyimide, polycarbonate, polyetheretherketone (PEEK), polystyrene (PS), polysulfone, and polyethersulfone (PES). Preferred thermoset elastomers include styrene-butadiene, polybutadiene (BR), ethylene-propylene, acrylonitrile-butadiene (NBR), polyisoprene, polychloroprene, silicone, fluorosilicone, urethanes, hydrogenated nitrile rubber (HNBR), polynorborene (PNR), butyl rubber (IIR) to include chlorobutyl (CIIR) and bromobutyl (BIIR), fluoroelastomers such as Viton® and Kalrez®, Fluorel™, and chlorosulfonated polyethylene. Preferred thermoplastic elastomer (TPE) categories include thermoplastic olefins (TPO) including those commercially available as Dexflex® and Indure®; elastomeric PVC blends and alloys; styrenic block copolymers (SBC) including styrene-butadiene-styrene (SBS), styrene-isoprene-styrene (SIS), styrene-ethylene/butylene-styrene (SEBS), and styrene-ethylene-propylene-styrene (SEPS), some commercially available SBCs include Kraton®, Dynaflex®, and Chronoprene™; thermoplastic vulcanizate (TPV, also known as dynamically vulcanized alloys) including those commercially available as Versalloy®, Santoprene®, and Sarlink®; thermoplastic polyurethane (TPU) including those commercially available as ChronoThane®, Versollan™, and Texrin®; copolyester thermoplastic elastomers (COPE) including those commercially available as Ecdel®; and polyether block copolyamides (COPA) including those commercially available as PEBAX®. Preferred metals for porous materials include stainless steel, aluminum, zinc, copper and its alloys. Preferred glass and ceramics for porous materials include quartz, borosilicate, aluminosilicate, sodiumaluminosilicate, preferably in the form of sintered particles or fibers derived from said materials. The foregoing list of preferred materials is referenced throughout this specification.

A preferred method of making macroporous plastic is by a process called sintering, wherein powdered or granular thermoplastic polymers are subjected to the action of heat and pressure to cause partial agglomeration of the granules and formation of a cohesive macroporous sheet or part. The macroporous material comprises a network of interconnected macropores that form a random tortuous path through the sheet. Typically, the void volume or percent porosity of a macroporous sheet is from 30 to 65% depending on the conditions of sintering although it may be greater or lesser than the stated range depending on the specific method of manufacturer. Due to surface tension, macroporous material can be tailored to repel or absorb liquids, but air and other gases can readily pass through. U.S. Patent No. 3,051,993 to Goldman discloses the details of making a macroporous plastic from polyethylene.

Porous plastic, including macroporous plastic, suitable for making a vent in accordance with preferred embodiments, can be manufactured in sheets or molded to specification and is available for purchase from a number of sources. Porex Corporation (Fairburn, Georgia, U.S.A.) is one such source, and provides porous plastic under the trademark, POREX®. Porous plastic sold under the name POREX® can be purchased in sheets or molded to specification from any one of the thermoplastic polymers previously described. The average porosity of such POREX® materials can vary from about 1 to 350 microns depending on the size of polymer granules used and the conditions employed during sintering. GenPore (Reading, Pennsylvania, U.S.A.) is another manufacturer of porous plastic products, with pore sizes ranging from 5 to 1000 microns. MA Industries Inc. (Peachtree City, Georgia, U.S.A.) also manufactures porous plastic products. Porvair Technology Ltd (Wrexham North Wales, U.K.) is another manufacturer of porous products supplying both porous plastic (range of 5 to 200um pore size under brand name Vyon™) and porous metal media (under brand name Sinterflo®).

The basic size, thickness and porosity of the plastic chosen to make a vent may be determined by calculating the amount of material that must pass through the vent in a given period of time (flow rate). The flow rate for a given area of vent is known as the flux rate. The flow and flux rates of a given macroporous plastic varies depending on factors including the pore size, percent porosity, and cross sectional thickness of the vent and is generally expressed in terms of fluid volume per unit time per unit area for flux rate and volume per unit time for flow rates. To achieve a sufficient degree of venting, the flow rate of the vent is such that the volume of air per minute that passes through the vent equals or exceeds the volume of beverage per minute that is removed from the container by drinking or dispensing. In the case of an infant, a flow rate of about 50 to 200ml/min of fluid delivery is sufficient to provide a pleasurable drinking experience, whereas for most adults under normal drinking conditions, a flow rate of about 250 to 5000 ml/min of fluid delivery is preferred. In a preferred embodiment, the combination of macroporous vent pore size, percent porosity, and thickness results in venting rates capable of providing on average about 50 to 5000ml/min fluid or beverage delivery rates out of the container, including about 75, 100, 200, 250, 300, 400, 500, 600, 700, 750, 800, 900, 1000, 1500, 2000, 2500, 3000, 3500, 4000 and 4500 ml/min including about 50 to 200ml/min for infants, about 100 to 500ml/min for toddlers, about 250 to 2500ml/min for children, and about 500 to 5000ml/min for young and mature adults. In a preferred embodiment, the flux of beverage delivered through a vented closure is about 50 to 5000 ml/min*cm², including about 75, 100, 200, 250, 300, 400, 500, 600, 700, 750, 800, 900, 1000, 1250, 1500, 1750, 2000, 2500, 3000, 3500, 4000 and 4500 ml/min*cm².

In common usage, "Macroporosity" generally refers to the overall void volume of a material or its macrostructure. The term "Macroporous" is generally used to classify a material's individual pores that are considered large in size. The term "Microporosity" generally refers to the individual pore sizes or distribution of pore sizes that constitute the microstructure of a porous material. The term "Microporous" is generally used to classify a material's individual pores that are considered small in size. For purposes of the disclosure herein, pore size (diameter) is classified according to the International Union of Pure and Applied Chemistry (IUPAC) Subcommittee of Macromolecular Terminology, definitions of terms drafted on February 26, 2002. This standard divides pore size classification into three categories: Microporous (< 0.002µm), Mesoporous (0.002 to 0.050µm) and Macroporous (>0.050µm). Also for the purposes of this disclosure herein, void volume will be discussed in terms of the "Percent Porosity" of the material.

Preferred porous materials include those in which the pores on opposite surfaces (what will become the interior and exterior surfaces) are interconnected such that the two sides are in communication with each other. Such interconnections are preferably not, however, straight through as to create a tubes or ports through which material passes; instead a network of pores creates a tortuous path for the liquid or gas to pass.

For a single layer vent, the porous materials are preferably macroporous with pore sizes greater than or equal to 0.05µm, preferably about 0.1 to 500µm, and about 0.5 to 10µm, including 0.25, 0.5, 1, 5, 15, 20, 40, 60, 80, 100, 150, 200, 250, 300, 350, 400, and 450 µm. In one embodiment, the vent materials used in conjunction have pore sizes between 0.1 and 100µm, preferably between 0.5 and 75µm. The percent porosity (percent open area) of the materials are preferably about 10 to 90%, including 30 to 75% or 50 to 70%, including 20%, 40%, 60%, and 80%. Preferred thicknesses of the porous materials include from 0.025 to 7mm, including between 1 and 3mm. Preferred thickness for vent materials include about 0.05 to 5mm and about 0.1 to 3.0mm, including 0.2, 0.3, 0.5, 0.7, 1.0, 1.25, 1.5, 1.75, 2.0, and 2.5mm. Other embodiments may have values for the above parameters that are above or below those set forth above. For the values set forth in this paragraph, as well as elsewhere in the specification, the stated ranges include as the values contained in between the values specifically mentioned. In other embodiments, materials can have one or more properties having values lying outside the disclosed ranges.

The vent material can be derived from plastic, elastomers, glass, metal, or combinations thereof. Some preferred matrix materials, including thermoplastic polymers, thermoset elastomers, thermoplastic elastomer, metals, glass and ceramics are as detailed above. Vent materials may be purchased from commercial sources, or they may be made according to a variety of techniques. U.S. Patent No. 4,076,656 to White et al. details one technique in which porogens are added to molten or dissolved materials, which can be leached out with a solvent, or extracted with supercritical fluids after the material sets and is in its final form. U.S. Patent No. 5,262,444 to Rusincovitch et al. details another technique to create porous material by introducing porogens that evolve into gases after processing a material, to leave behind a porous structure.

Single layer porous vent material is advantageously used to provide venting for hot liquid and food container closures such as those used for carry-out applications. These may include containers for hot liquids such as coffee, tea, chocolate, soups, gravies, and sauces. Low cost porous vent materials with low to medium air flux rates and high water intrusion pressures are well suited for this type of application. The porous vent material preferably does not substantially detract from the structural integrity of the closure. In another embodiment, porous venting materials with similar characteristics to the above mentioned materials are advantageously selected to provide venting for plasticware type food storage containers that may be disposable or reusable depending on the desired usage. The vented food containers are also suited for microwave heating environments, in which they will allow the food container to safely vent steam during the heating process. In microwavable embodiments, preferred porous materials are made from plastics including elastomers, as metal would be disadvantageous for microwave heating or reheating. Preferred plastics include high-density polyethylene (HDPE), ultra-high molecular weight polyethylene (UHMWPE), polypropylene (PP), polymethylpentene (PMP), polyetheretherketone (PEEK), poly(acrylonitrile-butadiene-styrene) (ABS) polyesters, polyvinyldichloride (PVDC) polyvinylfluoride (PVF), polyvinylidinefluoride (PVDF), polytetrafluoroethylene (PTFE), polyamides, polyethylenetetrafluoroethylene (ETFE), polyperfluoroalkoxyethylene (PFA), polyimide, polycarbonate. Preferred elastomers are of the thermoset type and include styrene-butadiene, polybutadiene (BR), ethylene-propylene, acrylonitrile-butadiene (NBR), polyisoprene, polychloroprene, silicone, fluorosilicone, urethanes, hydrogenated nitrile rubber (HNBR), polynorborene (PNR), butyl rubber (IIR) to include chlorobutyl (CIIR) and bromobutyl (BIIR), as well as other plastics referenced above.

The basic size, thickness and porosity of the plastic chosen to make a vent may be determined by calculating the amount of air that must pass through the vent in a given period of time (flow rate). The flow rate of a given macroporous plastic varies depending on factors including the pore size, percent porosity, and cross sectional thickness of the vent and is generally expressed in terms of fluid volume per unit time. To achieve a sufficient degree of venting, the flow rate of the vent should be such that the volume of air per minute that passes through the vent in or out of the container is sufficient to maintain the atmospheric pressure inside of the container in balance with the outside container pressure. In addition, to achieve a sufficient degree of venting during consumption from a hot beverage container, the flow rate of the vent should be such that the volume of ambient air per minute that passes through the vent into the container is sufficient to replace the volume of liquid consumed during the immediate time frame. Preferred flow rates are disclosed above and include about 10 to 3500ml/min or about 500 to 2500ml/min for venting of steam, between about 10 to 100ml/min for hot liquids to vent steam outside of the container, and about 50 to 1000ml/min including about 100 to 500ml/min for venting of air into hot beverage containers to aid consumption of the beverage. It should be noted that because of the interrelatedness of the concepts of flow rates and flux rates (a flux rate being a flow rate per unit area), these terms may be used somewhat interchangeably when referring to desired properties of a matrix material.

For laminated hydrophobic vent materials, the resultant properties of the final vent material will depend at least in part on the unique characteristics of each laminate that comprises the laminate. For example, a thin material with poor structural integrity, high water intrusion pressure, and high flux rate can be laminated to a thicker material with good structural integrity, low water intrusion pressure, and high flux rate to produce a vent material with high water intrusion pressure, high flux rate, and good structural integrity. In such an embodiment, preferred thin laminants have high water intrusion pressure and high flux rates, and are preferably derived from plastic, elastomers, metals, or ceramic materials including the specific materials mentioned hereinabove. Thin layers preferably range between about 20µm and 1000µm with average pore size preferably between about 0.5 and 350µm, including between about 5 and 150µm, and the percent porosity is preferably about 10 to 90%, including about 30 to 75%, and about 50 to 70%. The foregoing ranges are those used in connection with certain preferred embodiments. Use of materials having values outside the stated ranges if desirable for a particular application is contemplated.

The thin layers can be laminated to thicker layers using techniques familiar to those in the art. Thick laminants are preferably derived from plastic, elastomers, metals, or ceramic materials, including but not limited to the listing of preferred materials listed *supra.* Thickness preferably ranges from about 100 to 5000µm with average pore sizes preferably ranging from about 0.5 to 500µm. The percent porosity of the thick layer materials can range from about 10 to 90%, including between 30 to 75%, and between 50 to 70%.

Vent material may also be derived from porous materials made from blends. In a preferred embodiment, the porous materials comprise a fluorinated resin, including, but not limited to, polyvinylfluoride (PVF), polyvinylidinefluoride (PVDF), polytetrafluoroethylene (PTFE), polyethylenetetrafluoroethylene (ETFE), fluorinated ethylene propylene (FEP), polyperfluoroalkoxyethylene (PFA), and/or fluorinated additives such as Zonal®, blended with one or more selected polyolefin or other resins, including those selected from the series of polyethylenes (LLDPE, LDPE, MDPE, HDPE, UHMWPE) polypropylene, polyesters, polycarbonates, ABS, acrylics, styrene polymethylpentene (PMP), polybutylene terephthalate (PBT); polyethyleneterephthalate (PET), polyetheretherketone (PEEK), ethylenevinylacetate (EVA), polyacetal, poly(acrylonitrile-butadiene-styrene) (ABS), poly(acrylonitrile-styrene-acrylate) (AES), poly(acrylonirile-ethylene-propylene-styrene) (ASA), polyesters, polyacrylates, polymethacrylates polymethylmethacrylate (PMMA), polyvinylchloride (PVC), polyvinyldichloride (PVDC) nylon 6 (N6), polyamide, polyimide, polycarbonate, polystyrene, and polyethersulfone (PES). The resulting blends, including sintered blends, have porous structures with varying amounts of porosity, flexibility and mechanical strength determined predominately from the non-PTFE or other non-fluorinated resin, and high water intrusion pressures determined predominately from the fluorinated resin due to its preferential migration to the pore surface during the sintering process. The percent porosity, pore size, and thickness are preferably as noted above. Blended matrix materials may be purchased from commercial sources, or they may be made according to a variety of techniques. U.S. Patent No. 5,693,273 to Wolbrom details a process of cosintering to produce multi-porosity porous plastic sheets that can be derived from two or more polymeric resin materials and U.S. Patent No. 5,804,074 to Takiguchi et al. et al. details a process to produce a plastic filter by cosintering two or more polymeric resins in a molding process to produce filter parts.

Some porous materials are permeable to liquids. The rate of permeability is related to its liquid flux rate. The liquid flux rate is determined by factors including the pore size, percent porosity, surface tension, and cross sectional thickness. A favorable combination of these factors produces liquid flux rates capable of delivering beverage liquids from a container at suitable flow rates, including those described hereinabove which have been found provide a pleasurable drinking experience.

Porous materials can be constructed or engineered to be hydrophilic. Commodity plastic materials such as nylon, polysulfone, and the cellulosics, are available in hydrophilic grades. These hydrophilic materials can be milled into particles and sintered using techniques known to those familiar in the art to produce hydrophilic porous materials with high liquid flux rates. Porous hydrophilic plastic, including macroporous plastic, suitable for liquid beverage delivery in accordance with preferred embodiments, can be manufactured in sheets or molded to specification and is available for purchase from a number of sources. Porex Corporation (Fairburn, Georgia, U.S.A.) is one such source, and provides hydrophilic porous plastic under the trademark, POREX®. Porous plastic sold under the name POREX® can be purchased in sheets or molded to specification from any one of the thermoplastic polymers previously described. The average porosity of such materials can vary from about 1 to 350 microns depending on the size of polymer granules used and the conditions employed during sintering. GenPore (Reading, Pennsylvania, U.S.A.) is another manufacturer of hydrophilic porous plastic products, with pore sizes ranging from 5 to 1000 microns. MA Industries Inc. (Peachtree City, Georgia, U.S.A.) also manufactures hydrophilic porous plastic products. Porvair Technology Ltd (Wrexham North Wales, U.K.) is another manufacturer of hydrophilic porous products supplying both porous plastic (range of 5 to 200um pore size under brand name Vyon™) and porous metal media (under brand name Sinterflo®). Porous hydrophilic fiber materials preferably range in pore size from 20 to 120µm with percent porosity ranging from 25 to 80 for the pore volume. Moreover, hydrophobic porous materials, including many of those referenced hereinabove, can be rendered hydrophilic by one or more treatment processes familiar to those skilled in the art including, but not limited to, plasma etching, chemical etching, impregnation with wetting agents, or application of hydrophilic coatings. In addition, a masking process can be used in conjunction with one or more treatment processes to selectively pattern a hydrophobic porous material with regions of hydrophilicity with high liquid flux rates. The patterned materials can advantageously be incorporated into beverage container closures to provide additional control over regulating the flow of fluid from inside to outside the container during consumption. In one embodiment, the patterned porous material is used to provide a rotatable flow selector integral to the beverage closure. Techniques used to render hydrophilic materials more hydrophobic may also be used to render hydrophobic materials more hydrophilic.

A porous vent can be fabricated for assembly into a beverage closure or container, for example, by die cutting or stamping out a disc or ring-shaped geometry from a sheet of macroporous material. The porous vent may also be sinter molded with a suitable process and mold design to yield the final vent geometry in one process. The sinter molding process produces less waste than stamping from sheets, and can be economical depending on the number of parts and tooling costs. Other porous part geometries can be similarly and readily produced with these two techniques, as well as other suitable techniques as may be known or apparent to those skilled in the art to yield components suitable for container closures and containers.

In preferred embodiments, the containers and container closures described herein deliver generally aqueous liquids having surface tensions of approximately 40-75 mN/m, or the range of surface tensions found in most beverages. Although preferred embodiments described herein relate to delivery of beverages, the concepts and closures described herein may be used in the delivery of any fluid.

In the context of this specification, "vent matrix", "vent material" and similar terms refer to porous materials which allow for easy passage of air while generally avoiding passage of bulk liquid and thus provide venting capabilities. In a vent matrix used with an aqueous liquid, the air flux rate of the vent matrix is high, the water or liquid flux rate is low, and it has a high water intrusion pressure.

### Vented Containers

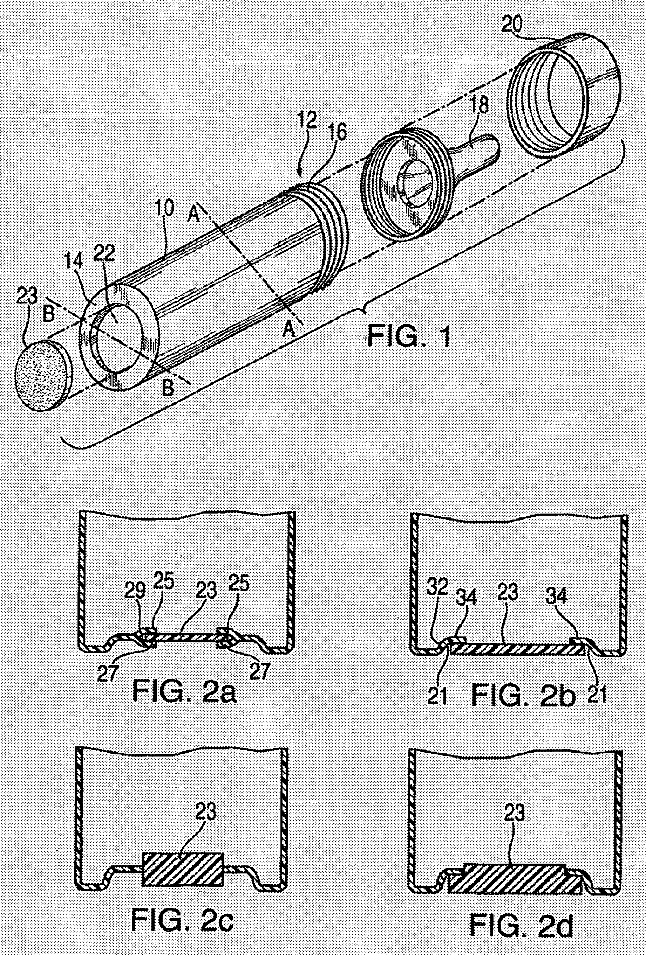

As shown in Figure 1, a vented container is depicted in the form of a baby bottle. The baby bottle comprises an elongated cylindrical bottle 10 having an open end 12 and a partially closed end 14. The bottle body is preferably formed from a thermoplastic polymer, including, but not limited to, polypropylene, polyethylene or polycarbonate by processes known in the art such as blowmolding or injection molding. The bottle body is formed with a threaded lip 16 at its open end 12 so that an elastomeric nipple 18 can be clamped against the top of the bottle by a threaded ring 20 that is screwed onto the threaded lip 16 of the bottle. The partially closed end 14 of the bottle body is formed with a hole 22 for receiving a vent 23. The vent 23 is made from macroporous plastic and is preferably secured in the hole by one of the methods discussed below.

Once the macroporous vent is obtained, the vent can be secured to the plastic bottle body by any one of a number of methods. The vent may be molded into a cavity that is formed in a wall of the bottle as the bottle is being injection molded (i.e. insert molded). With reference to Figure 2a, an example is shown wherein the hole-forming detail molded into the bottle wall comprises an inner and outer lip 25 and 27 defining a circular cavity 29 having an inside dimension that corresponds to the outside dimension of the vent 23. Prior to injection molding, the vent 23 would be positioned in the injection mold such that when molten plastic is injected into the mold, the lip detail will form in the bottle wall around the edges of the vent such that a leak-proof seal is created between the bottle wall and the vent with the vent being permanently secured in place.

In a second example, the bottle body is blow molded or injection molded with a hole whereby the hole-forming detail in the bottle wall comprises a circular depression 21 as shown in Figure 2b. A vent disc 23, dimensioned to fit snugly against the sides 32 and bottom 34 of the depression 21, is secured in place using means such as ultrasonic sealing or welding as are known in the art. In the case of welding, the edges of the vent and bottle wall that are to be welded together are subjected to a heat source until melted, and then the edges are butted together and clamped in place until cool. Low temperature heating suitable for welding is preferably accomplished using one of the following: plastics hot-air gun, hot-air blower, infrared heat lamp, radiant tube, wire, or ribbon; or spin-welding techniques.

During any welding, heating or molding process, one should preferably limit the application of heat to the edges of the vent so that the porous characteristics of the vent are not substantially altered anywhere except at the edges of the vent.

The vent can also be secured in place using a sealant or adhesive. The type of sealant used depends on the ability of the sealant to bond with or penetrate the pores of the plastic. One example uses PVC and/or ABS cement to mechanically bond PP to PVC, styrene or ABS. In certain applications, two-part epoxy systems or silicone may be used to secure the vent in place. One consideration is that the adhesive be chemically compatible with the vent material and the other material(s) being bonded.

With reference to Figure 2c and Figure 2d, the vent can also be formed as a plug 23 that can be inserted into a hole 22 formed in the wall of the bottle during blow molding or injection molding of the bottle body. In one example, the plug is formed from PTFE and the plug 23 has an outside diameter slightly larger than diameter of the hole 22. In order to insert the plug into the hole, the plug is subjected to low temperature, such as by exposing the plug to liquid nitrogen. The cold temperature causes the plug to shrink enough that the plug can be inserted in the hole. Upon warming, the plug expands to its original size, thus plugging the hole and forming a water-tight seal between the bottle wall and the plug. The plug could also be press fit into the bottle.

One may also use one of the methods described above to secure the vent to a threaded, plastic screw cap similar to the threaded ring 20 used to clamp the nipple onto the open end of the bottle. In this case, the bottle would comprise an elongated tube threaded at each end. The nipple could be clamped to one end of the bottle using the threaded ring and a threaded screw cap provided with a macroporous vent could be threaded on the other end of the bottle body. In a related embodiment, a snap-fit cap may be used in place of the screw cap to secure the vent.

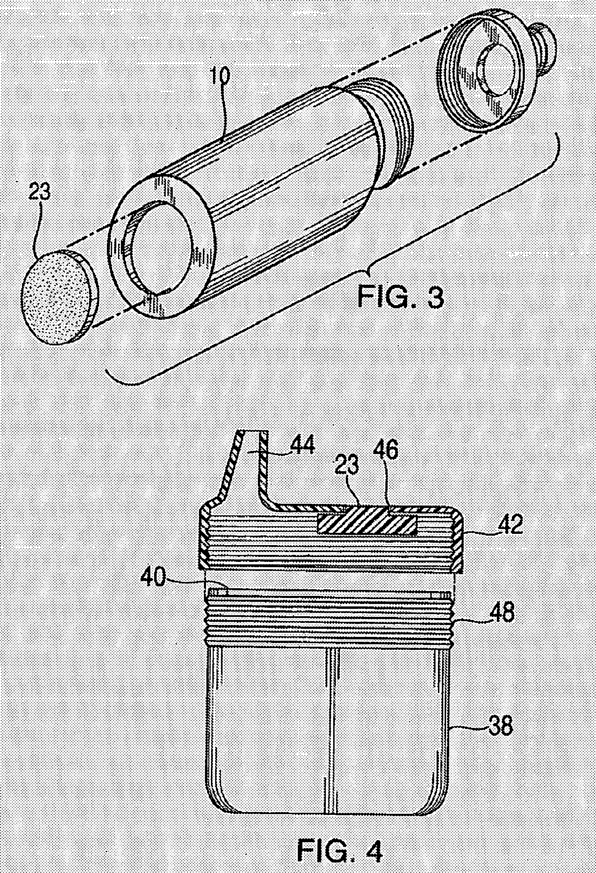

The same methods used to secure the vent to a baby bottle body may also be used to secure the vent to the plastic bodies of other kinds of beverage bottles or containers. As before, the bottle or container is preferably formed from plastic by processes such as blowmolding or injection molding. Examples of these types of bottles or containers include soda-pop bottles, water bottles, sports bottles and canteens. With reference to Figure 3, a water bottle 10 is shown with a vent 23 secured in the base.

It is also possible to use one of the methods described above to secure the vent to a plastic cover for a drinking cup. With reference to Figure 4, a drinking cup 38 is threaded at its open end 40. A plastic cover 42 is formed with a rigid drinking spout 44 to one side, a hole forming detail 46 to the other side, and threads 48 for clamping the cover to the cup. The vent 23 is secured in the hole 46 using one of the above-described securing methods. Both the cup and the cover are preferably formed from plastic by processes known in the art such as blowmolding or injection molding.

The previously discussed methods used to secure a vent to a plastic bottle body can also be used to secure a vent to a glass or metal container. For example, the bottle can be molded with a hole-forming detail as previously described and the plastic vent secured therein using sealant or the cold-shrink method. An embodiment in which the vent is secured using a screw cap or snap-cap may also be used with glass or metal containers.

In an alternative embodiment, the vent may be formed from metal or glass by sintering powdered glass or metal under selected conditions of heat and pressure causing partial agglomeration of the granules and formation of a cohesive macroporous substrate. Depending on the conditions chosen, an average porosity of 7 to 350 microns and a void volume of 30 to 65% can be achieved. The glass or metal is preferably rendered hydrophobic either prior to the molding process or subsequent to the molding process using surface modification agents such as organosilanes so as to reduce unwanted leakage of generally aqueous contents. The size, thickness and porosity of a vent may be determined as previously described by calculating the flux rate or flow rate. The sintering conditions and mold dimensions can then be conformed to yield a vent having the desired properties. The glass or metal vent can be secured to a glass, metal, or plastic container using the methods discussed above.

Several embodiments described herein and those illustrated herein utilize a disk-shaped vent. While a disc shape may be preferred for ease of manufacturing and functional efficiency, it is possible to use vents of different shapes and geometries, e.g., oval or rectangular and any such alternate shape is presently contemplated. Preferably the shape of the vent does not prevent the vent from being secured in a leak-proof manner such as by using one of the securing methods disclosed above or equivalent methods.

Although the examples described with reference to Figure 2 locate the vent in the closed end of the bottle, the vent or multiple vents can just as easily be located along the sidewall of the bottle, and such embodiments are contemplated. The venting material is preferably constructed from hydrophobic macroporous materials, that negate the requirement of moving parts to control venting. The vented closure may be secured to any type of beverage container including plastic, glass bottle, and cans. In the disclosure herein, any of a variety of means and methods may be used to secure or attach a closure to a container. Such means and methods include fittings which are threaded, press fit, snap fit, interference fit, and/or compression fit; adhesives applied to one or more surfaces of the container or closure; welding, including ultrasonic welding; and/or other closure means and methods known in the art. The term "secured" as used herein in reference to the connecting or attachment of a closure to a container, is a broad term, used in its ordinary sense, and includes removable, non-removable (i.e. cannot be removed without disrupting the structure of the closure and/or container), and unitary (e.g. a single, monolithic piece, or the functional equivalent thereof) modes. The term "containers" as used herein is a broad term, used in its ordinary sense, and includes bottles, cans, canteens, jars, and other vessels suitable for holding and/or dispensing liquids. Containers may be made of any suitable material. Also the terms "connected to" and "attached to" are broad terms, used in their ordinary senses, to describe the relationship between two or more parts, include where the parts are removably attached, non-removably attached, adhered such as by adhesives, unitary construction of the two parts, attachment by threaded or press-fit connections, insert molded together, and the like.

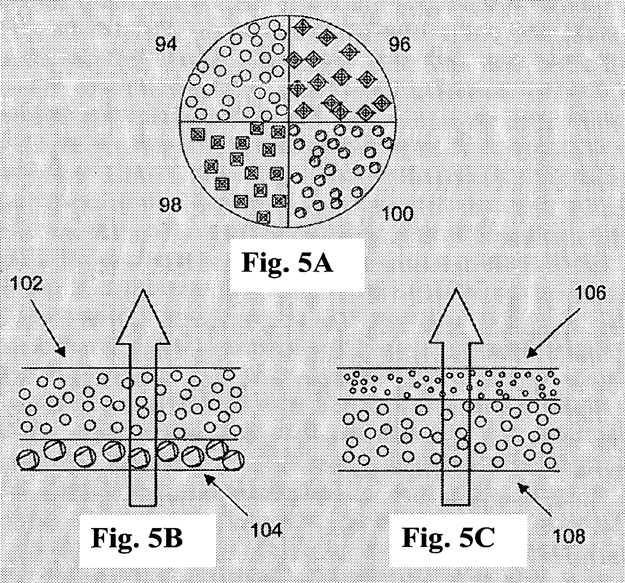

Figures 5A-5C depict various arrangements of porosity in accordance with the invention in one or more planes. Porous matrix combinations are used to obtain properties that are generally not possible with single materials. Figure 5A shows a single layer of porous material that has been patterned to yield regions of discrete porous properties. The patterning is produced as such by using a suitable masking technique followed by chemical, plasma, or coating treatments. Regions 94-100 are made to differ in hydrophilicity, which alters the materials' flux rates and corresponding water intrusion pressures. The construction of vented closures using this feature is advantageous in providing improved fluidic control during consumption.

Figures 5B and 5C depict embodiments of 2-ply laminate constructions of porous matrix materials. In figure 5B, a thin layer of relatively hydrophobic porous material (104) has been laminated to a thicker layer of porous material (102) to provide a single matrix with properties derived from both (102) and (104). The direction of flow is shown by the arrow, and the flow is from the thin layer through the thick layer. The construction shown in figure 5B is advantageous for constructing porous hydrophobic vents for container enclosures, where high water intrusion pressures and high air flux rates are needed. In figure 5C, a thin porous layer of material (106) is shown laminated to a thick porous layer of material (108) with resulting flow properties indicated by arrow, which shows flow from the thicker material through the thinner. The construction shown in figure 5C is advantageous for constructing porous flow control matrixes where high liquid flux rates and low water intrusion pressures are needed.

### Vented and Partitioned Multi-Component Beverage Closure/Container Systems

Porous materials can be advantageously incorporated into beverage containers to provide a novel mixing system for multi-component beverages. Typically these beverage containers are constructed from partitioned or multi-cavity bodies containing two or more separate fluid compartments. This novel mixing system is particularly well suited for multicomponent beverage components capable of spontaneous carbonation when mixed. In one embodiment, a hydrophobic porous material with low water intrusion pressure and high liquid flux rate is layered to a thicker region of hydrophilic porous material with high liquid flux rate. The beverage container cavities and partitions are sealed at the top by the porous laminant material. Additional hydrophobic vent material may also be provided preferably in the beverage closure body to provide vacuum elimination during consumption that also affords uniformly mixed liquid components exiting from the random and tortuous porous path of interconnected pores into the spout. Hydrophobic porous vent material can be provided in the container body if desired. In a related embodiment, a straw can be readily integrated into the above delivery system that provides multi-component mixing.

### Packaging Configuration for Carbonated Beverage Vented Closure

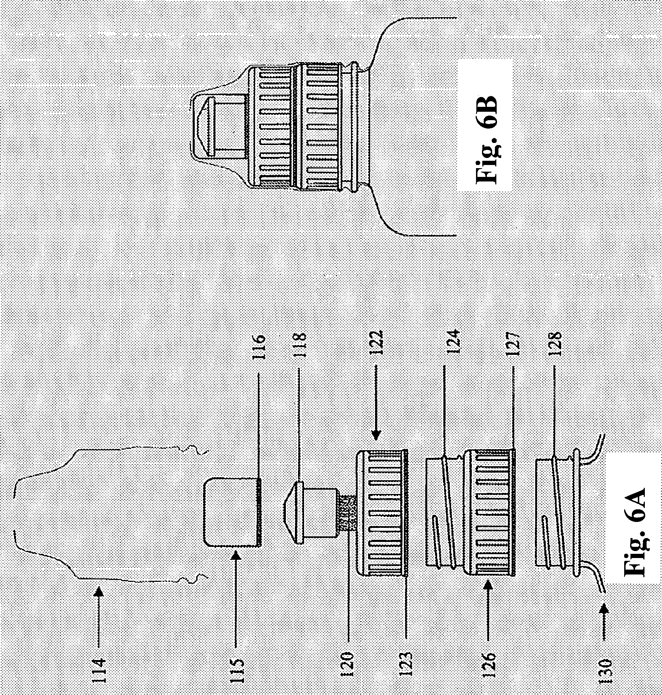

For carbonated beverages, the vented closures can be readily packaged by the bottler along with the container without loss of carbonation. In one embodiment of such closure, as shown in Figures 6A and 6B, a break-away design is incorporated into the mid-section of a container closure assembly that provides separation of the vented closure from the disposable primary storage closure. Once liberated the vented closure is secured to the container body before use. Figure 6A is an exploded view of the final packaging configuration for a preferred vented closure containing a recloseable, preferably telescopic, spout (118), porous vent matrix (120), threaded vented closure body (122) with complimentary threads to opening (124) of primary closure body (126). In Figure 6A a double closure stack is shown shrink-wrapped (114) with protective capsule (115) placed over spout (118) and secured to the top of the vented closure (122) with a break-away, twist, or pull-off mechanism (116). The vented closure body (122) is joined to the primary closure (126) with a break-away, twist, or pull-off mechanism (123). The primary closure (126) is secured to the container body (130) by a threading mechanism, and has provisions for a break-away, twist, or pull-off mechanism (127) to ensure integrity of the contents. The primary closure is engineered to support the maintenance of carbonation within the beverage after packaging and during long-term storage. In use, the primary closure assembly is initially separated from the container body first, followed by separation of the vented closure from the primary. Then, the primary closure is removed and discarded followed by securing the vented closure to the container opening. In an alternate embodiment, the primary closure is eliminated and a secondary seal, such as a peel away foil seal, covers the opening of the bottle or the inside of the vented closure body. Figure 6B shows the final packaging configuration with a protective shrink-wrap over the double closure stack that can also be used to ensure product integrity in addition to the mechanism shown in (127).

### Multifunctional Carbonated Beverage Closure System

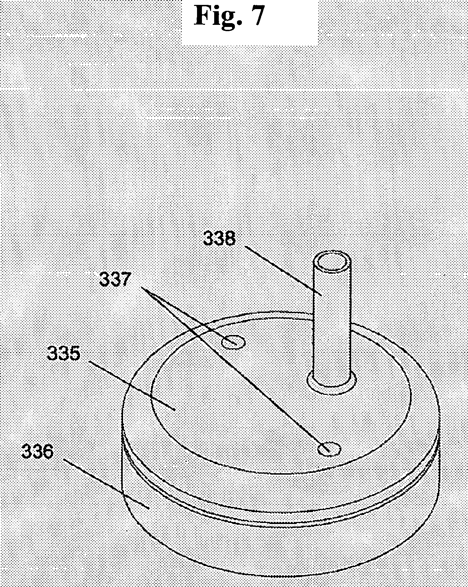

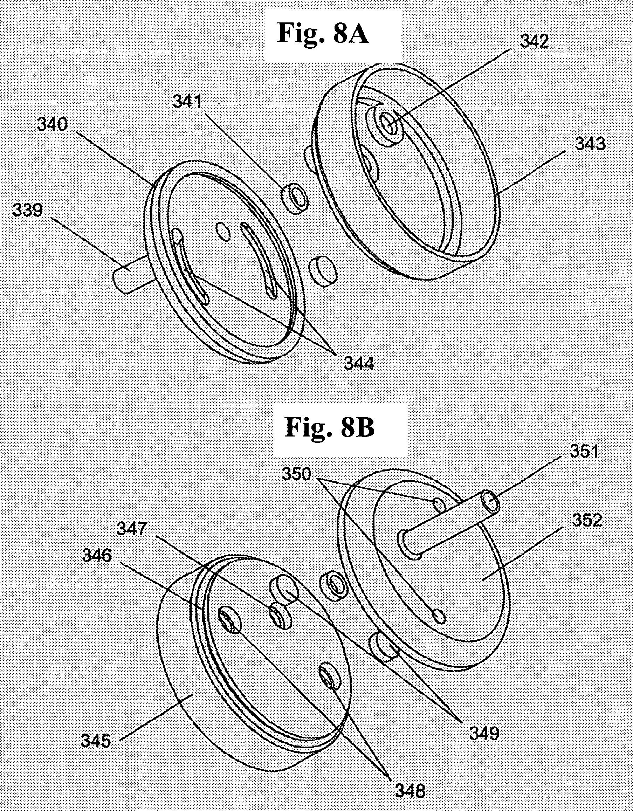

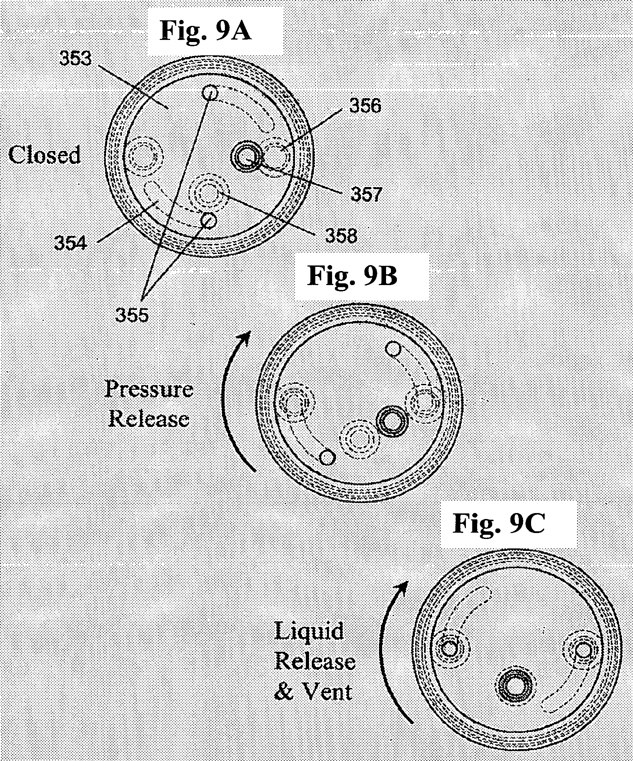

Figures 7, 8A and 8B, 9A, 9B, and 9C show a multifunctional carbonated beverage closure system suitable for the primary closure of carbonated beverages upon bottling, through shipping and long term storage by the consumer. By rotation of the cap out of its storage position, the carbonated closure functions to safely release excessive pressure through the porous matrix, thereby containing liquid and any foam within the beverage container. Both foam and liquid are advantageously prevented from passage through the hydrophobic porous matrix to the outside. By rotating the cap once more, the vents remain open while a liquid pathway is aligned through the closure to advantageously allow consumption or delivery of beverage. In Fig. 7, the closure top (335) is shown with two vent holes (337) and a fluid delivery spout (338) protruding outwards in relationship to the closure base (336). Not shown are alternative configurations that allow for closing and resealing of the spout. Figures 8A & 8B show the exploded view of the carbonated beverage closure with one of the two vent holes (342) shown in the closure base (343), and the same two vent holes (350) in the closure top (352). The liquid ring seal (341) provides a leak free path for the beverage fluid to flow from the container through the spout (339), and is located in the recess (347) of the closure base (345). The hydrophobic porous vent discs (349) allow for the equilibration (pressure or vacuum) of the container with the outside atmosphere, and are located within the recesses (348) of the closure base (345). In an alternative configuration (not shown) the hydrophobic discs are integral to the closure top (340). Venting grooves (344) allow for the passage of gasses between the closure base (345) and the closure top (340). A rotational snap groove (346) is located at the top edge of the closure base (343). The groove provides a compressive seal that is maintained between the closure top (352) and closure base (345) during rotation of the closure top to various positions shown in figures 9A through 9C. Figure 9A shows the top view of the closure in the closed position with the closure top (353) shown with a transparent view revealing the venting grooves (354), the liquid ring seal (358) in between the vent holes (355), and the spout (357) near the hydrophobic porous disc (356). Figure 9B shows the closure having been rotated to a second position allowing the hydrophobic porous venting materials (disks) to align with the venting grooves, thereby allowing safe release of pressure from the container without liquid loss from the container. Figure 9C shows the closure having been rotated to a third position which allows venting to continue while enabling the passage of beverage liquid through the aligned spout.

Various methods and techniques are described above. Of course, it is to be understood that not necessarily all objectives or advantages described may be achieved in accordance with any particular solution described herein.

Furthermore, the skilled artisan will recognize the interchangeability of various features from different solutions. Similarly, the various features and steps discussed above, as well as other known equivalents for each such feature or step, can be mixed and matched by one of ordinary skill in this art. Merely the principle design of the invention and some other solutions are described above whereby the scope of inventive idea is presented in the accompanying claims.

## Claims

1. A closure for dispensing fluids from a container (130), comprising:
a pair of telescopically coupled first and second members (118, 122) cooperatively defining a fluid path;
said first member (122) attached to a base (126) adapted to be secured to the container;
wherein the base and/or the first member include one or more sections of a porous vent material (120) which allows passage of gases and inhibits bulk passage of liquid,
**characterised in that** said porous vent material has at least two parts (94, 96, 98, 100, 102, 104, 106, 108) of discrete properties for control of fluid flow, said two parts being in the form of a lamination or surface pattern, and
wherein said at least two parts (94, 96, 98, 100, 102, 104, 106, 108) of discrete properties comprise at least a first part which is poorer in structural integrity, higher in water intrusion pressure, and higher in flux rate compared to the at least second part.

2. A closure according to Claim 1, wherein the vent material (120) comprises plastic, metal, ceramic and/or glass.

3. A closure according to Claim 1, wherein the vent material (120) comprises a hydrophobic material.

4. A closure according to Claim 1, wherein the vent material (120) comprises a plastic material having a high water intrusion pressure.

5. A closure according to Claim 1, wherein the porous vent material (120) provides sufficient venting to allow a substantially continuous liquid flux rate from the closure without creating a substantial pressure differential across the closure.

6. A closure according to Claim 1, wherein the porous vent material (120) provides sufficient venting to allow a substantially continuous liquid flux rate from the closure of at least about 50 ml/min/cm².

7. A closure according to Claim 1, wherein the porous vent material (120) provides sufficient venting to allow a substantially continuous liquid flux rate from the closure of at least about 500 ml/min/cm².

8. A closure according to Claim 1, wherein the first and second members (118, 122) are generally tubular.

9. A closure according to Claim 1, wherein the fluid path is opened to allow fluid flow out of a container by moving the second member (118) relative to the first member (122).

10. A closure according to Claim 1, wherein the base (126) includes female threads.

11. A closure according to Claim 10 in combination with a container, wherein the container has a neck with external threads (128) adapted to cooperate with the threads on the base to attach the closure to the container.

12. A closure according to Claim 1, wherein the one or more sections of porous vent material (120) are disposed on the first member.

13. A closure according to Claim 12, wherein the porous vent material (120) is covered by the second member (118) when the closure is in a closed position and exposed to air when the closure is in an open position.

14. A closure according to Claim 1, wherein the first member (122) and the base (126) are unitary.

15. A closure according to Claim 1, wherein the vent material (120) has an average porosity of from 0.1 to 500 microns.

16. A closure according to Claim 1, wherein the vent material (120) is disc shaped and has a thickness between 20 to 5000 microns.

17. A closure according to Claim 1, wherein the vent material (120) is secured to the base and/or the first member by any one of heat welding, injection molding, sealant, sonic welding, and insertion.

18. A closure according to Claim 1, wherein the vent material (120) is secured to the base and/or the first member to create a leak-proof seal between the vent material and the base and/or the first member.

19. A closure according to Claim 1, wherein carbonation and negative pressure are both relieved through the vent material (120).

20. A closure according to Claim 1, wherein a flux rate of the vent material (120) permits a volume of air per minute that passes through the vented material to equal or exceed a volume of beverage per minute that is removed from the container (130).

21. A closure according to Claim 1, wherein said porous vent material (120) comprises at least two laminations of discrete thickness.

22. A closure according to Claim 1, wherein said porous vent material (120) is in the form of a laminate which is a selectively patterned hydrophobic porous material having regions of hydrophilicity with high liquid flux rates.

23. A container (130) comprising the closure of any of the preceding claims.

## Patentansprüche

1. Verschluss zur Abgabe bzw. Dosierung von Flüssigkeiten aus einem Behälter (130) mit einem ersten und einem zweiten Teil (122, 118), das teleskopisch mit dem anderen Teil verbunden ist und die zusammen einen Flüssigkeitspfad definieren, wobei das erste Teil (122) an einem an den Behälter anschließbaren Basisteil (126) anbringbar ist, wobei das Basisteil bzw. das erste Teil einen oder mehrere Abschnitte aus porösem Belüftungsmaterial (120) umfasst bzw. umfassen, das einen Durchtritt von Gasen ermöglicht und einen größeren Durchtritt von Flüssigkeit verhindert, **dadurch gekennzeichnet, dass** das genannte poröse Belüftungsmaterial mindestens zwei Teile (94, 96, 98, 100, 102, 104, 106, 108) unterschiedlicher Eigenschaften zur Kontrolle des Flüssigkeitsstroms besitzt, wobei die genannten zwei Teile einen Schichtaufbau oder ein Oberflächenmuster aufweisen, und wobei die mindestens zwei Teile (94, 96, 98, 100, 102, 104, 106, 108) unterschiedlicher Eigenschaften wenigstens ein erstes Teil enthalten, das im Vergleich zum zweiten Teil eine geringere Festigkeit, einen höheren Wassereindringdruck sowie eine höhere Durchströmrate aufweist.

2. Verschluss gemäß Anspruch 1, wobei das Belüftungsmaterial (120) aus Kunststoff, Metall, Keramik bzw. Glas besteht.

3. Verschluss gemäß Anspruch 1, wobei das Belüftungsmaterial (120) aus einem wasserabweisenden Material besteht.

4. Verschluss gemäß Anspruch 1, wobei das Belüftungsmaterial (120) aus einem Kunststoffmaterial mit hohem Wassereindringdruck besteht.

5. Verschluss gemäß Anspruch 1, wobei das Belüftungsmaterial (120) eine ausreichend große Belüftung ermöglicht, so dass eine im wesentlichen kontinuierliche Flüssigkeitsdurchströmrate durch die Öffnung gewährleistet ist, ohne dass im Bereich der Öffnung ein erhebliches Druckgefälle verursacht wird.

6. Verschluss gemäß Anspruch 1, wobei das Belüftungsmaterial (120) eine ausreichend große Belüftung ermöglicht, so dass ein im wesentlichen kontinuierlicher Flüssigkeitsdurchsatz durch die Öffnung von mindestens 50 ml/min/cm² gewährleistet ist.

7. Verschluss gemäß Anspruch 1, wobei das Belüftungsmaterial (120) eine ausreichend große Belüftung ermöglicht, so dass ein im wesentlichen kontinuierlicher Flüssigkeitsdurchsatz durch die Öffnung von mindestens 500 ml/min/cm² gewährleistet ist.

8. Verschluss gemäß Anspruch 1, wobei das erste und zweite Teil (122, 118) im wesentlichen rohrförmig ist.

9. Verschluss gemäß Anspruch 1, wobei die Flüssigkeit aus einem Behälter austreten kann, indem das zweite Teil relativ zum ersten Teil bewegt wird.

10. Verschluss gemäß Anspruch 1, wobei das Basisteil (126) ein Innengewinde aufweist.

11. Verschluss gemäß Anspruch 10 in Kombination mit einem Behälter, wobei der Behälter einen Hals mit einem Außengewinde (128) aufweist, das beim Anbringen des Verschlusses am Behälter mit dem Gewinde am Basisteil zusammenwirkt.

12. Verschluss gemäß Anspruch 1, wobei ein oder mehrere Abschnitt/e des porösen Materials (120) zur Belüftung an dem ersten Teil angeordnet ist bzw. sind.

13. Verschluss gemäß Anspruch 12, wobei das poröse Belüftungsmaterial (120) bei geschlossenem Verschluss von dem zweiten Teil (118) bedeckt und bei geöffnetem Verschluss der Luft ausgesetzt ist.

14. Verschluss gemäß Anspruch 1, wobei das erste Teil (122) und das Basisteil (126) einteilig ausgebildet sind.

15. Verschluss gemäß Anspruch 1, wobei das poröse Belüftungsmaterial (120) eine durchschnittliche Porosität von 0,1 bis 500 Mikron aufweist.

16. Verschluss gemäß Anspruch 1, wobei das poröse Belüftungsmaterial (120) scheibenförmig ist und eine Dicke von 20 bis 5000 Mikron aufweist.

17. Verschluss gemäß Anspruch 1, wobei das poröse Belüftungsmaterial (120) am Basisteil und/oder am ersten Teil durch Schweißen, Spritzguss, Versiegeln, Ultraschall-Schweißen oder Insertion befestigt ist.

18. Verschluss gemäß Anspruch 1, wobei das poröse Material (120) so am Basisteil und/oder am ersten Teil befestigt ist, dass eine auslaufsichere Abdichtung zwischen dem Belüftungsmaterial (120) und dem Basisteil und/oder dem ersten Teil erreicht wird.

19. Verschluss gemäß Anspruch 1, wobei sowohl Kohlensäure als auch Unterdruck durch das Belüftungsmaterial (120) entweichen können.

20. Verschluss gemäß Anspruch 1, wobei eine Durchströmrate des Belüftungsmaterials (120) gewährleistet, dass das durch das Material zur Belüftung pro Minute durchtretende Luftvolumen dem Volumen des Getränks entspricht oder das Volumen des Getränks übersteigt, das dem Behälter (130) entnommen wird.

21. Verschluss gemäß Anspruch 1, wobei das poröse Belüftungsmaterial (120) aus mindestens zwei Schichten verschiedener Dicke besteht.

22. Verschluss gemäß Anspruch 1, wobei das poröse Belüftungsmaterial (120) als Schichtstoff ausgebildet ist, der wahlweise aus wasserabweisendem porösem Material mit eine hohe Flüssigkeitsströmung zulassenden, hydrophilen Bereichen besteht.

23. Behälter (136), der mit einem Verschluss gemäß einem der vorgenannten Ansprüche ausgestattetet ist.

## Revendications

1. Une fermeture pour la distribution de fluides à partir d'un container (130) comprenant :
un premier et un second membre assemblés de manière télescopique définissant coopérativement une conduite de fluide ;
ledit premier membre (122) attaché à une base (126) adaptée pour être fixée sur le container ;
dans lequel la base et/ou le premier corps inclus une ou plusieurs sections d'un matériel d'orifice poreux (120) qui permet le passage de gaz et limite le passage de liquide,
**Caractérisé en ce que** ledit matériel d'orifice poreux comprend au moins deux parties (94, 96, 98, 100, 102, 104, 106, 108) de propriétés distinctes pour le contrôle de l'écoulement de fluides, lesdites parties ayant la forme d'un laminage ou d'une surface modèle, et
dans lequel au moins deux parties (94, 96, 98, 100, 102, 104, 106, 108) de propriétés distinctes comprennent au moins une première partie qui est plus pauvre en intégrité structurelle, plus élevée en intrusion de pression d'eau et plus élevée en taux de flux en comparaison avec la deuxième partie.

2. Une fermeture selon la revendication 1, dans laquelle le matériel d'orifice poreux (120) comprend du plastic, du métal, de la céramique et/ou du verre.

3. Une fermeture selon la revendication 1, dans laquelle le matériel d'orifice poreux (120) comprend une matière hydrophobe.

4. Une fermeture selon la revendication 1, dans laquelle le matériel d'orifice poreux (120) comprend une matière plastique ayant une haute intrusion en pression d'eau.

5. Une fermeture selon la revendication 1, dans laquelle le matériel d'orifice poreux (120) fournit suffisamment de ventilation pour permettre substantiellement un taux continu de flux liquide à partir de la fermeture sans créer une substantielle pression différentielle à travers la fermeture.

6. Une fermeture selon la revendication 1, dans laquelle le matériel d'orifice poreux (120) fournit suffisamment de ventilation pour permettre substantiellement un taux continu de flux liquide à partir de la fermeture d'au moins environ 50 ml/min/cm2.

7. Une fermeture selon la revendication 1, dans laquelle le matériel d'orifice poreux (120) fournit suffisamment de ventilation pour permettre substantiellement un taux continu de flux liquide à partir de la fermeture d'au moins environ 500 ml/min/cm2.

8. Une fermeture selon la revendication 1, dans laquelle le premier et le second membre (118, 122) sont généralement tubulaires.

9. Une fermeture selon la revendication 1, dans laquelle la conduite de fluide est ouverte pour permettre au fluide de s'écouler en dehors du container en déplaçant le second membre (118) par rapport au premier membre (122).

10. Une fermeture selon la revendication 1, dans laquelle la base (126) inclus des filetages femelles.

11. Une fermeture selon la revendication 10 en combinaison avec un container, dans laquelle le container a un goulot comprenant des filetages extérieurs (128) adaptés pour coopérer avec les filetages sur la base pour fixer la fermeture au container.

12. Une fermeture selon la revendication 1, dans laquelle une ou plusieurs sections du matériel d'orifice poreux (120) sont disposés sur le premier membre.

13. Une fermeture selon la revendication 12, dans laquelle le matériel d'orifice poreux (120) est couvert par le second membre (118) lorsque la fermeture est en position fermée et exposé à l'air lorsque la fermeture est en position ouverte.

14. Une fermeture selon la revendication 1, dans laquelle le premier membre (122) et la base (126) sont unitaires.

15. Une fermeture selon la revendication 1, dans laquelle le matériel d'orifice (120) a une moyenne de porosité comprise entre 0.1 et 500 microns.

16. Une fermeture selon la revendication 1, dans laquelle le matériel d'orifice (120) a la forme d'un disque et a une épaisseur comprise entre 20 et 5000 microns.

17. Une fermeture selon la revendication 1, dans laquelle le matériel d'orifice (120) est fixé à la base et/ou au premier membre par l'un des moyens de thermosoudure, moulage par injection, mastic, soudure par ultrasons et vibrations et insertion.

18. Une fermeture selon la revendication 1, dans laquelle le matériel d'orifice (120) est fixé à la base et/ou au premier membre pour créer une étanchéité entre le matériel d'orifice et la base et/ou le premier membre.

19. Une fermeture selon la revendication 1, dans laquelle la carbonisation et la pression négative sont toutes deux relevées à travers le matériel d'orifice.

20. Une fermeture selon la revendication 1, dans laquelle le taux de flux du matériel d'orifice (120) permet un volume d'air par minute qui passe à travers le matériel d'orifice pour être égal ou excéder un volume de boisson par minute qui est supprimé du container (130).

21. Une fermeture selon la revendication 1, dans laquelle le matériel d'orifice poreux (120) comprend au moins deux laminages d'épaisseurs distinctes.

22. Une fermeture selon la revendication 1, dans laquelle ledit matériel d'orifice poreux (120) a la forme d'un laminage qui est un matériel poreux hydrophobe à motifs ayant sélectivement des zones d'hydrophilicité avec un taux élevé de flux liquide.

23. Un container (136) comprenant la fermeture de l'une des précédentes revendications.
